# EUROPEAN PATENT SPECIFICATION

(11) **EP 1 143 942 B1**
(45) Date of publication and mention of the grant of the patent: **13.08.2003**
(21) Application number: 00914167.2
(22) Date of filing: 23.03.2000
(51) Int. Cl.: A61K 31/00, A61P 25/16, A61P 25/28

(54) **USE OF GABA-B RECEPTOR LIGANDS FOR THE MANUFACTURE OF MEDICAMENTS FOR THE TREATMENT OF NEURODEGENERATIVE DISEASES**
VERWENDUNG VON GABA-B REZEPTOR LIGANDEN ZUR HERSTELLUNGEN VON MEDIKAMENTEN ZUR BEHANDLUNG VON NEURODEGENERATIVEN ERKRANKUNGEN
UTILISATION DES LIGANDS DU RECEPTEUR GABA-B POUR L'OBTENTION D'UN MEDICAMENT DESTINE A TRAITER DES MALADIES NEURODEGENERATIVES

(30) Priority: 25.03.1999 GB 9906882
(43) Date of publication of application: 17.10.2001
(73) Proprietor: Novartis AG, 4056 Basel (CH); Novartis Pharma GmbH, 1230 Wien (AT)
(72) Inventor: BERNASCONI, Raymond, CH-4104 Oberwil (CH); OTTEN, Uwe, CH-4051 Basel (CH)
(74) Representative: Grubb, Philip William
(86) International application number: EP0002605
(87) International publication number: WO00057862

(56) References cited:
- EP-A- 0 319 479
- EP-A- 0 399 949
- EP-A- 0 463 969
- EP-A- 0 569 333
- WO-A-97/46675
- WO-A-98/28313
- WO-A-98/58939
- FR-A- 2 722 192
- US-A- 3 978 216
- HEESE K ET AL: "GABA B RECEPTOR ANTAGONISTS ELEVATE BOTH MRNA AND PROTEIN LEVELS OFTHE NEUROTROPHINS NERVE GROWTH FACTOR (NGF) AND BRAIN-DERIVED NEUROTROPHIC FACTOR (BDNF) BUT NOT NEUROTROPHIN-3 (NT-3) IN BRAIN AND SPINAL CORD OF RATS" NEUROPHARMACOLOGY,PERGAMON PRESS, OXFORD,GB, vol. 39, 2000, pages 449-462, XP000938344 ISSN: 0028-3908
- LAL, SUMEER ET AL: "Baclofen is cytoprotective to cerebral ischemia in gerbils" NEUROCHEM. RES. (1995), 20(2), 115-19 , XP000981832
- KULINSKII, V. I. ET AL: "Effect of bilateral destruction of the subretrofacial area on receptor mechanisms of neuroprotective effect of GABAergic agents" BULL. EXP. BIOL. MED. (1998), 125(2), 139-141 , XP000981867
- YU, ZAIFANG (1) ET AL: "Mechanism of colchicine impairment on learning and memory, and protective effect of CGP-36742 in mice." BRAIN RESEARCH, (1997) VOL. 750, NO. 1-2, PP. 53-58. , XP000981780
- J.E.F. REYNOLDS: "MARTINDALE, The Extra Pharmacopoeia" 1996 , ROYAL PHARMACEUTICAL SOCIETY , LONDON XP002160961 page 1515 -page 1516 Baclofen
- LEES A J ET AL: "BACLOFEN IN PARKINSONS DISEASE." J NEUROL NEUROSURG PSYCHIATRY, (1978) 41 (8), 707-708. , XP000981829
- "GABA B RECEPTOR ANTAGONISTS: FROM SYNTHESIS TO THERAPEUTIC APPLICATIONS" TRENDS IN PHARMACOLOGICAL SCIENCES,ELSEVIER TRENDS JOURNAL, CAMBRIDGE,GB, vol. 14, 1993, pages 391-393, XP000946102 ISSN: 0165-6147 cited in the application
- POTASHNER, S. J. ET AL: "Kainate-enhanced release of D-[3H]aspartate from cerebral cortex and striatum: reversal by baclofen and pentobarbital" J. NEUROCHEM. (1983), 40(6), 1548-57 , XP000981821
- SMITH, G. D. (1) ET AL: "Increased sensitivity to the antinociceptive activity of (racemic)-baclofen in an animal model of chronic neuropathic, but not chronic inflammatory hyperalgesia." NEUROPHARMACOLOGY, (1994) VOL. 33, NO. 9, PP. 1103-1108. , XP000986543
- ENNA, S. J. ET AL: "Regulation of neurokinin-1 receptor expression by GABAB receptor agonists" LIFE SCI. (1998), 62(17/18), 1525-1530 , XP000983025
- GEORGE, B. ET AL: "Protective effects of GABAergic drugs and other anticonvulsants in lithium-pilocarpine-induced status epilepticus." METHODS AND FINDINGS IN EXPERIMENTAL AND CLINICAL PHARMACOLOGY, (1996) VOL. 18, NO. 5, PP. 335-340. , XP000981822

## Description

The present invention relates to a new pharmaceutical use of GABA_{B} receptor antagonists.

More particularly the present invention relates to the use of GABA_{B} receptor antagonists for the manufacture of medicaments for increasing neurotrophin levels in the central nervous system (CNS).

The neurotrophins are known to be involved in neuronal survival, the growth and differentiation of synaptic efficacy and plasticity (Lo, D.C., 1996, Neuron 15, 979-981; Lessmann V., 1998, Gen. Pharmac. 31, 667-674). For example, animal studies have shown that neurotrophins can reduce or prevent age-related axotomy or neurotoxin-induced neuronal loss or reduced function in a variety of brain regions and nerve growth factor (NGF) significantly improves cognition in aged rats (Femandez C. et al., 1995, Mol. Chem. Neuropathol. 24, 241-244). Moreover, one case report has shown that the intracerebroventricular (i.c.v.) infusion of NGF in a patient with Alzheimer's disease (AD) resulted in an increase in nicotine binding in the frontal and temporal cortices and in a persistent increase in cortical blood flow combined with a significant improvement of verbal episodic memory (Olson L. et al., 1992, J. Neural Transm. [P-D Sect.] 4, 79-95). These preliminary results have recently been confirmed by Jönhagen et al. Dement. Geriatr. Cogn. Disord. 1998: 9, 246-257, who showed that i.c.v. infusion of NGF in three patients with AD for three months leads to improvement of CNS effects including upregulation of nicotinic receptors in the brain, an increased cortical blood flow, a decrease in slow-wave EEG activity and an improved performance in cognitive tests. The occurrence of two negative side effects (back pain and marked weight reduction) required to stop this clinical trial after three months. These reports suggested that NGF counteracts the cholinergic deficit in AD. The possibility that naturally occurring degeneration of the basal forebrain system, such as that seen in AD, may be inhibited by exogenous neurotrophin administration, opens up the field of neurotrophin therapy for neurodegenerative diseases. However, the clinical utility of neurotrophic factors is limited by the fact that they do not cross the blood-brain barrier and are easily metabolised by peptidases when administered peripherally (Barinaga, M. 1994, Science 264, 772-774). Thus, the need of invasive neurosurgical procedures (i.c.v. delivery catheter) severely restricts the utility of neurotrophins such as NGF and brain-derived neurotrophic factor (BDNF) as therapeutic agents for the treatment of behavioral and cognitive deficits related to aging and neurodegenerative diseases. Systemic treatment with trophic factors also causes serious side effects (Barinaga, see above).

Thus, there is a need to find means of stimulating endogenous neurotrophin synthesis in the brain by administration of substances that cross the blood brain barrier.

Kulinskii et al. (1998; Bulletin of Experimental Biology and Medicine, No 2, Biophysics and Biochemistry, page 139-141) describes that GABA_{B} receptor agonists produce marked neuroprotective effect during total brain ischemia.

In accordance with the present invention it has now surprisingly been found that GABA_{B} receptor antagonists enhance expression of neurotrophin mRNA and protein levels in various brain regions. Moreover it has been found that GABA_{B} receptor antagonists exert said activity with a remarkably long duration of action.

GABA_{B} antagonists are known for example from USP 5,051,524 or USP 5,332,729. Specific GABA_{B} antagonists include for example 3-{1(S)-[3-(cyclohexylmethyl)hydroxyphosphinyl)-2(S)-hydroxy-propylamino]ethyl}benzoic acid (hereinafter compound A), 3-{1(R)-[3-(cyclohexylmethyl)hydroxyphosphinyl-2(S)-hydroxy-propylamino]ethyl}benzoic acid (hereinafter compound B) and 3-aminopropyl-(n-butyl)-phosphinic acid, and their salts. For a review on GABA_{B} receptor antagonists and their therapeutic applications, see for example Bittiger, et al., TiPS 1993: 14, 391-393.

The effects of GABA_{B} receptor antagonists on the expression of neurotrophins is indicated in studies performed for example as follows:

Male adult Wistar rats (10-12 weeks) are killed by decapitation. The brains are removed, rapidly dissected with nuclease-free instruments on an ice-cold metal-plate, transferred to sterile cryotubes, and immediately shock-frozen by immersion in liquid nitrogen. Tissues are stored at -80°C until further processing. Samples for the measurement of neurotrophin protein levels are prepared according to Lüesse et al., Exp. Brain Res. 1998: 119, 1-8. The homogenates are centrifuged at 12,500 g for 60 min, and the supernatants are used for NGF, BDNF and neurotrophin-3 (NT-3) quantification using specific ELISA assays.

Total cellular RNA is isolated according to the TRIZOL®-protocol (Gibco BRL, Eggenstein) as described by Lüesse et al. (see above).

Before being subjected to RT-PCR, the RNA extracts are supplemented with internal control RNA and optimized as described in detail by Heese K. et al., 1998, Neural Notes III, 21-23. Total RNA of each sample is first reverse-transcribed into cDNA which in turn is subjected to PCR amplification using primers specific for NGF and BDNF as described by Heese K. et al., (see above).

For the quantification of neurotrophin transcripts, the ratios of densitometric scores for NGF or BDNF and S12 PCR products are calculated. Data are means ± SEM of three independent experiments, each done in duplicate. N = 4 to 6 for each group.

To measure the immunoreactive NGF into the cortex, hippocampus and spinal cord of rats, a two-site ELISA is used (Weskamp, G. and Otten, U., 1987, J. Neurochem. 48, 1779-1786). Anti-β (2.5S, 7S) NGF and anti-β (2.5S, 7S) NGF-β-gal (clone 27/21) (Boehringer Mannheim) are applied, and the NGF content in the samples is determined by comparison with an NGF standard curve (absorbance measurement at 595 nm using an ELISA reader, Dynatech MR 700). For quantification of BDNF and NT-3 levels, specific immunoassay systems are used according to the manufacturer's (Promega) protocols but modified by Heese, K. and Otten, U., 1998, J. Neurochem. 70, 699-707. Statistical evaluation of results is performed by applying analysis of variance, and the statistical error is the SEM. Recovery is 80% using recombinant mouse NGF as internal standard.

In these tests, GABA_{B} receptor antagonists at doses of about 0.1 to about 600 mg/kg i.p. significantly increase NGF- and BDNF-mRNA in the cortex, hippocampus and spinal cord 6 to 24 hours after treatment and significantly increase NGF- and BDNF-protein levels in said regions 12 to 72 hours after treatment.

For example with compound B on administration of 1 and 6 mg/kg i.p., a 3 to 4-fold increase of NGF mRNA and a 2.0 to 4.0-fold increase of BDNF mRNA is induced in said regions 6 and 24 hours after treatment, whereas with compound A on administration of 3 and 10 mg/kg i.p., a 2.0 to 2.5-fold increase of NGF mRNA and a 2 to 3-fold increase of BDNF mRNA is induced in said regions 6 and 24 hours after treatment. Similarly with compound B, on administration of 1 mg/kg i.p., a 1.5 to 2-fold increase of NGF protein (peak values at 24-48 hours after treatment) and a 2 to 2.5-fold increase of BDNF protein (peak values at 72 hours) is induced in said regions.

GABA_{B} receptor antagonists are therefore useful in the treatment of any condition responsive to an increase of neurotrophin levels in the CNS. Particularly GABA_{B} receptor antagonists are useful for the treatment of conditions responsive to an increase of NGF and BDNF.

Such conditions include neurodegenerative diseases such as amyotrophic lateral sclerosis and Parkinson's disease.

For the above-mentioned indications the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 600 mg/kg body weight. In larger mammals, for example humans, an indicated daily dosage is in the range from about 1 to about 2000 mg of a compound for use according to the invention conveniently administered, for example, in divided doses up to five times a day.

The present invention accordingly relates to the use of a GABA_{B} receptor antagonist in the manufacture of a medicament for the treatment of the above-mentioned conditions.

For use according to the invention, the GABA_{B} receptor antagonist may be administered as single active agent or in combination with other active agents, in any usual manner, e.g. orally, for example in the form of tablets or capsules, or parenterally, for example in the form of injection solutions or suspensions.

More particularly, the present invention provides the use of a GABA_{B} receptor antagonist for the manufacture of a pharmaceutical composition for the treatment of Parkinson's disease and amyotrophic lateral sclerosis.

## Claims

1. The use of a GABA_{B} receptor antagonist for the manufacture of a pharmaceutical composition for the treatment of Parkinson's disease and amyotrophic lateral sclerosis.

## Patentansprüche

1. Verwendung eines GABA_{B}-Rezeptorantagonisten zur Herstellung einer pharmazeutischen Zusammensetzung zur Behandlung der Parkinsonkrankheit und der amyotrophen Lateralsklerose.

## Revendications

1. Utilisation d'un antagoniste du récepteur GABA_{B} pour la fabrication d'une composition pharmaceutique destinée au traitement de la maladie de Parkinson et de la sclérose latérale amyotrophique.
